# EUROPEAN PATENT SPECIFICATION

(11) **EP 2 421 555 B1**
(45) Date of publication and mention of the grant of the patent: **29.06.2016**
(21) Application number: 10719953.1
(22) Date of filing: 23.04.2010
(51) Int. Cl.: C07K 14/44, A61K 39/00

(54) **NUCLEIC ACID MOLECULE**
NUKLEINSÄUREMOLEKÜL
MOLÉCULE D'ACIDE NUCLÉIQUE

(30) Priority: 23.04.2009 GB 0906906
(43) Date of publication of application: 29.02.2012
(73) Proprietor: The University Of York, York, Yorkshire YO10 5DD (GB); University Of Edinburgh, Edinburgh EH8 9YL (GB)
(72) Inventor: KAYE, Paul, York YO10 5DD (GB); SMITH, Deborah, York YO10 5DD (GB); LACEY, Charles, York YO10 5DD (GB); AEBISCHER, Anton, 13302 Berlin (DE)
(74) Representative: Docherty, Robert Charles
(86) International application number: PCT/GB2010/000815
(87) International publication number: WO 2010/122310

(56) References cited:
- WO-A2-2008/064181
- STÄGER S ET AL: "Immunization with a recombinant stage-regulated surface protein from Leishmania donovani induces protection against visceral leishmaniasis", JOURNAL OF IMMUNOLOGY, AMERICAN ASSOCIATION OF IMMUNOLOGISTS, US, vol. 165, 1 January 2000 (2000-01-01), pages 7064-7071, XP002296035, ISSN: 0022-1767
- MORENO ET AL: "Immunization with H1, HASPB1 and MML Leishmania proteins in a vaccine trial against experimental canine leishmaniasis", VACCINE, ELSEVIER LTD, GB LNKD- DOI:10.1016/J.VACCINE.2007.05.010, vol. 25, no. 29, 28 June 2007 (2007-06-28) , pages 5290-5300, XP022133788, ISSN: 0264-410X
- ALCE TIMOTHY M ET AL: "Expression of hydrophilic surface proteins in infective stages of Leishmania donovani", MOLECULAR AND BIOCHEMICAL PARASITOLOGY, vol. 102, no. 1, 30 July 1999 (1999-07-30) , pages 191-196, XP002600954, ISSN: 0166-6851
- STÄGER S ET AL: "Natural antibodies and complement are endogenous adjuvants for vaccine-induced CD8+ T-cell responses", NATURE MEDICINE, NATURE PUBLISHING GROUP, NEW YORK, NY, US LNKD- DOI:10.1038/NM933, vol. 9, no. 10, 1 October 2003 (2003-10-01), pages 1287-1292, XP002296036, ISSN: 1078-8956

## Description

The invention relates to non-naturally occurring synthetic gene(s) and their use in a vaccine in providing prophylatic and therapeutic treatment of leishmaniasis.

The evolutionarily divergent, insect vector borne protozoan parasites of the order Kinetoplastidae cause a range of human and animal diseases, including leishmaniasis (a broad spectrum of infections caused by *Leishmania* species), Chagas' disease (*Trypanosoma cruzi*) and African sleeping sickness (*T. brucei*). These infections are of increasing prevalence in developing countries where mortality is often affected by poor access to health care. Many of the drugs available to treat these diseases are expensive, difficult to administer or toxic. No effective vaccines are available.

Leishmaniasis threatens many millions of people in around 88 countries and results in both a high level of death in those infected and retarded social/economic development of countries in which the disease is endemic. The disease has a number of symptoms depending on the infecting Leishmania species. For example, the cutaneous form of the disease is caused by *Leishmania aethiopica, L. mexicana, L.major, and L.tropica*; the mucocutaneous form by *L.braziliensis* and the visceral form by *L.chagasi, L.donovani* and *L.infantum.* The vector that transmits the disease is a sand fly, one of many species of the genus *Phlebotomus spp* [e.g. *Phlebotomus ariasi, P. pernicosus*] and *Lutzomyia spp* [e.g. *Lutzomyia longipalpis*]. The domestic reservoirs of leishmaniasis are dogs which can be asymptomatic and therefore both dogs that present with the disease and those that do not can be infectious when bitten by a sand fly. Cats can also harbour *Leishmania* but are considered secondary carriers. There is therefore a need to control infection not only in humans but also in animal species that act as reservoirs.

The chemotherapeutic control of leishmaniasis is unsatisfactory due to severe side effects, lack of efficacy and expense. Current drugs include pentavalent antimony, pentamide, pyrazolopyrimidines, amphotericin B, aminosidine, paromomycin and miltefosine. In some cases, although the disease can be controlled in dogs, they remain infectious. At present no effective vaccine is available although attempts to develop protective and therapeutic vaccines are known.

For example, the use of attenuated whole *Leishmania* organisms is disclosed in WO2005/021030. The vaccine uses live mutated *Leishmania* organisms that have a targeted disruption of the centrin gene. The centrin protein is found in the centrosome and functions in the movement of centrioles/basal bodies. *Leishmania* organisms that are deficient in centrin show inhibited cell growth. In WO98/44943 is described a further example of an attenuated Leishmania vaccine. The vaccine includes live *Leishmania* which carry deletions in cysteine proteases which lack disease manifestation. In WO99/51264 the use of cysteine proteases *per se* is described in subunit vaccine compositions to provide protective immunity. A subunit vaccine approach is described in CA2, 540, 736 which utilized 34 secreted *Leishmania major* antigens to provoke an immune response in mice. Similar subunit vaccine approaches are described in WO2008/064181; WO2007/121184; WO2005/049806; and EP1615663B1.

This disclosure relates to the creation of synthetic genes and their use in the vaccination of leishmaniasis.

According to an aspect of the invention there is provided a nucleic acid molecule that encodes a non-natural polypeptide comprising dominant leishmanial antigens.

According to an aspect of the invention there is provided a polypeptide encoded by a nucleic acid molecule according to the invention.

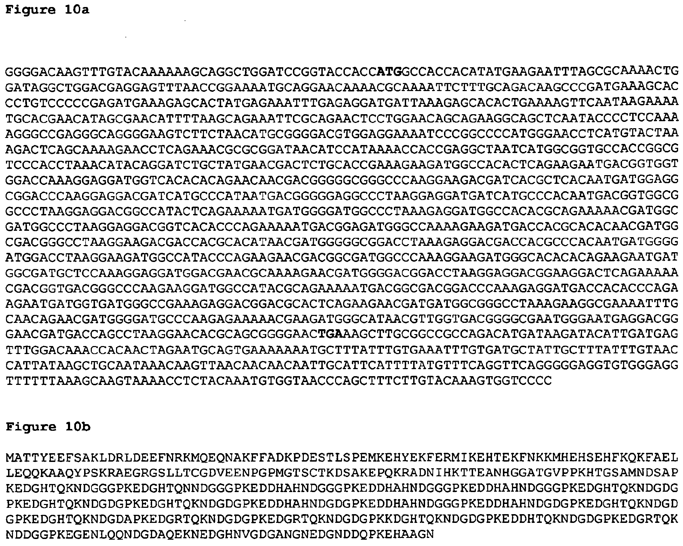

According to an aspect of the invention there is provided a nucleic acid molecule comprising a nucleotide sequence selected from the group consisting of:
(i) a nucleic acid molecule consisting of the nucleotide sequence as represented in Figure 10a;
(ii) a nucleic acid molecule comprising nucleotide sequences that are degenerate as a result of the genetic code to the nucleotide sequence defined in (i) and encodes an amino acid sequence as represented in Figure 10b.

Hybridization of a nucleic acid molecule occurs when two complementary nucleic acid molecules undergo an amount of hydrogen bonding to each other. The stringency of hybridization can vary according to the environmental conditions surrounding the nucleic acids, the nature of the hybridization method, and the composition and length of the nucleic acid molecules used. Calculations regarding hybridization conditions required for attaining particular degrees of stringency are discussed in Sambrook et al., Molecular Cloning: A Laboratory Manual (Cold Spring Harbour Laboratory Press, Cold Spring Harbor, NY, 2001); and Tijssen, Laboratory Techniques in Biochemistry and Molecular Biology-Hybridization with Nucleic Acid Probes Part I, Chapter 2 (Elsevier, New York, 1993). The Tₘ is the temperature at which 50% of a given strand of a nucleic acid molecule is hybridized to its complementary strand. The following is an exemplary set of hybridization conditions and is not limiting:

**Very High Stringency (allows sequences that share at least 90% identity to hybridize)**

| | |
|---|---|
| Hybridization: | 5x SSC at 65°C for 16 hours |
| Wash twice: | 2x SSC at room temperature (RT) for 15 minutes each |
| Wash twice: | 0.5x SSC at 65°C for 20 minutes each |

**High Stringency (allows sequences that share at least 80% identity to hybridize)**

| | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |

**Low Stringency (allows sequences that share at least 50% identity to hybridize)**

| | |
|---|---|
| Hybridization: | 6x SSC at RT to 55°C for 16-20 hours |
| Wash at least twice: | 2x-3x SSC at RT to 55°C for 20-30 minutes each. |

In a preferred embodiment of the invention there is provided a nucleic acid molecule comprising or consisting of a nucleotide sequence as represented in Figure 10a.

According to a further aspect of the invention there is provided a polypeptide encoded by a nucleic acid molecule according to the invention.

In a preferred embodiment of the invention said polypeptide is a variant polypeptide and comprises the amino acid sequence represented in Figure 10b, which sequence has been modified by deletion, addition or substitution of at least one amino acid residue.

A variant polypeptide may differ in amino acid sequence by one or more substitutions, additions, deletions, truncations that may be present in any combination. Among preferred variants are those that vary from a reference polypeptide by conservative amino acid substitutions. Such substitutions are those that substitute a given amino acid by another amino acid of like characteristics. The following non-limiting list of amino acids are considered conservative replacements (similar): a) alanine, serine, and threonine; b) glutamic acid and aspartic acid; c) asparagine and glutamine d) arginine and lysine; e) isoleucine, leucine, methionine and valine and f) phenylalanine, tyrosine and tryptophan. Most highly preferred are variants that retain or enhance the same biological function and activity as the reference polypeptide from which it varies.

According to an aspect of the invention there is provided a transcription cassette comprising: a nucleic acid molecule according to the invention operably linked to a promoter adapted for transcription of the nucleic acid molecule associated therewith.

In a preferred embodiment of the invention said promoter is a constitutive promoter.

In an alternative preferred embodiment of the invention said promoter is a regulatable promoter; preferably an inducible promoter and/or a tissue/cell specific promoter.

"Promoter" is an art recognised term and, for the sake of clarity, includes the following features which are provided by example only. Enhancer elements are *cis* acting nucleic acid sequences often found 5' to the transcription initiation site of a gene (enhancers can also be found 3' to a gene sequence or even located in intronic sequences). Enhancers function to increase the rate of transcription of the gene to which the enhancer is linked. Enhancer activity is responsive to *trans* acting transcription factors which have been shown to bind specifically to enhancer elements. The binding/activity of transcription factors (please see Eukaryotic Transcription Factors, by David S Latchman, Academic Press Ltd, San Diego) is responsive to a number of physiological/environmental cues. Promoter elements also include so called TATA box and RNA polymerase initiation selection sequences which function to select a site of transcription initiation. These sequences also bind polypeptides which function, *inter alia,* to facilitate transcription initiation selection by RNA polymerase.

In a preferred embodiment of the invention said promoter is a skeletal muscle specific promoter.

Muscle specific promoters are known in the art. For example, WO0009689 discloses a striated muscle preferentially expressed gene and cognate promoter, the SPEG gene.

EP1072680 discloses the regulatory region of the myostatin gene. The gene shows a predominantly muscle specfic pattern of gene expression. US5795872 discloses the use of the creatine kinase promoter to achieve high levels of expression of foreign proteins in muscle tissue. The muscle specific gene Myo D also shows a pattern of expression restricted to myoblasts. Further examples are disclosed in WO03/074711 which is incorporated by reference.

Preferably said constitutive promoter is selected from the group consisting of: Cytomegalovirus (CMV) promoter, β-globin RSV enhancer/promoter phosphoglycerate kinase (mouse PGK) promoter, alpha-actin promoter, SV40 promoter EF-1α promoter, ubiquitin promoter, transcription factor A (Tfam) promoter.

According to an aspect of the invention there is provided a vector comprising a nucleic acid molecule according to the invention.

In a preferred embodiment of the invention said vector is an expression vector adapted for expression of said nucleic acid molecule encoding a polypeptide according to the invention; preferably said nucleic acid molecule is operably linked to at least one promoter sequence.

Adaptations also include the provision of selectable markers and autonomous replication sequences which facilitate the maintenance of said vector in either the eukaryotic cell or prokaryotic host. Vectors which are maintained autonomously are referred to as episomal vectors. Episomal vectors are desirable since these molecules can incorporate large DNA fragments (30-50kb DNA). Episomal vectors of this type are described in WO98/07876. Adaptations which facilitate the expression of vector encoded genes include the provision of transcription termination/polyadenylation sequences. This also includes the provision of internal ribosome entry sites (IRES) which function to maximise expression of vector encoded genes arranged in bi-cistronic or multi-cistronic expression cassettes. Expression control sequences also include so-called Locus Control Regions (LCRs). These are regulatory elements which confer position-independent, copy number-dependent expression to linked genes when assayed as transgenic constructs. LCRs include regulatory elements that insulate transgenes from the silencing effects of adjacent heterochromatin, Grosveld et al., Cell (1987), 51: 975-985. Regulatory elements also include 2A sequences; see Luke et al J Gen Virology [2008] 89 1036-1042; Osbourne et al Mol Therapy [2005] 12 [3]: 569-74.

There is a significant amount of published literature with respect to expression vector construction and recombinant DNA techniques in general. Please see, Sambrook et al (1989) Molecular Cloning: A Laboratory Manual, Cold Spring Harbour Laboratory, Cold Spring Harbour, NY and references therein; Marston, F (1987) DNA Cloning Techniques: A Practical Approach Vol III IRL Press, Oxford UK; DNA Cloning: F M Ausubel et al, Current Protocols in Molecular Biology, John Wiley & Sons, Inc. (1994).

The use of viruses or "viral vectors" as therapeutic agents is well known in the art. Additionally, a number of viruses are commonly used as vectors for the delivery of exogenous genes. Commonly employed vectors include recombinantly modified enveloped or non-enveloped DNA and RNA viruses, preferably selected from *retroviridae baculoviridiae, parvoviridiae, picornoviridiae, herpesveridiae, poxviridae, adenoviridiae, or picornnaviridiae.* Chimeric vectors may also be employed which exploit advantageous elements of each of the parent vector properties (See e.g., Feng, et al. (1997) Nature Biotechnology 15:866-870). Such viral vectors may be wild-type or may be modified by recombinant DNA techniques to be replication deficient, conditionally replicating or replication competent. Preferred vectors are derived from retroviral genomes [e.g. lentivirus] or are adenoviral based.

Viral vectors may be conditionally replicating or replication competent. Conditionally replicating viral vectors are used to achieve selective expression in particular cell types while avoiding untoward broad spectrum infection. Examples of conditionally replicating vectors are described in Pennisi, E. (1996) Science 274:342-343; Russell, and S.J. (1994) Eur. J. of Cancer 30A(8):1165-1171. Additional examples of selectively replicating vectors include those vectors wherein a gene essential for replication of the virus is under control of a promoter which is active only in a particular cell type or cell state such that in the absence of expression of such gene, the virus will not replicate. Examples of such vectors are described in Henderson, et al., United States Patent No. 5,698,443 issued December 16, 1997 and Henderson, et al.; United States Patent No. 5,871,726 issued February 16, 1999 the entire teachings of which are herein incorporated by reference.

Additionally, the viral genome may be modified to include inducible promoters which achieve replication or expression only under certain conditions. Examples of inducible promoters are known in the scientific literature (See, e.g. Yoshida and Hamada (1997) Biochem. Biophys. Res. Comm. 230:426-430; lida, et al. (1996) J. Virol. 70(9):6054-6059; Hwang, et al. (1997) J. Virol 71(9):7128-7131; Lee, et al. (1997) Mol. Cell. Biol. 17(9):5097-5105; and Dreher, et al. (1997) J. Biol. Chem 272(46); 29364-29371.

According to an aspect of the invention there is provided a vaccine composition comprising a nucleic acid molecule or part thereof according to the invention.

According to an aspect of the invention there is provided a vaccine composition comprising a polypeptide or part thereof according to the invention.

In a preferred embodiment of the invention said composition includes a carrier and/or optionally an adjuvant.

In a preferred embodiment of the invention said adjuvant is selected from the group consisting of: cytokines selected from the group consisting of GMCSF, interferon gamma, interferon alpha, interferon beta, interleukin 12, interleukin 23, interleukin 17, interleukin 2, interleukin 1, TGF, TNFα, and TNFβ.

In a preferred embodiment of the invention said adjuvant is a Toll Like Receptor [TLR] agonist.

In an embodiment of the invention said TLR agonist is selected from CpG oligonucleotides, flagellin, monophosphoryl lipid A, poly I:C and derivatives thereof.

In a preferred embodiment of the invention said adjuvant is a bacterial cell wall derivative such as muramyl dipeptide (MDP) and/or trehelosedimycolat (TDM).

An adjuvant is a substance or procedure which augments specific immune responses to antigens by modulating the activity of immune cells. Examples of adjuvants include, by example only, agonistic antibodies to co-stimulatory molecules, Freunds adjuvant, muramyl dipeptides, liposomes. An adjuvant is therefore an immunomodulator. A carrier is an immunogenic molecule which, when bound to a second molecule augments immune responses to the latter. The term carrier is construed in the following manner. A carrier is an immunogenic molecule which, when bound to a second molecule augments immune responses to the latter. Some antigens are not intrinsically immunogenic yet may be capable of generating antibody responses when associated with a foreign protein molecule such as keyhole-limpet haemocyanin or tetanus toxoid. Such antigens contain B-cell epitopes but no T cell epitopes. The protein moiety of such a conjugate (the "carrier" protein) provides T-cell epitopes which stimulate helper T-cells that in turn stimulate antigen-specific B-cells to differentiate into plasma cells and produce antibody against the antigen. Helper T-cells can also stimulate other immune cells such as cytotoxic T-cells, and a carrier can fulfil an analogous role in generating cell-mediated immunity as well as antibodies. Certain antigens which lack T-cell epitopes, such as polymers with a repeating B-cell epitope (e.g. bacterial polysaccharides), are intrinsically immunogenic to a limited extent. These are known as T-independent antigens. Such antigens benefit from association with a carrier such as tetanus toxoid, under which circumstance they elicit much stronger antibody responses.

According to a further aspect of the invention there is provided a nucleic acid molecule according to the invention for use in the manufacture of a vaccine.

According to a further aspect of the invention there is provided a polypeptide according to the invention for use in the manufacture of a vaccine.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

An embodiment of the invention will now be described by example only and with reference to the following figures:

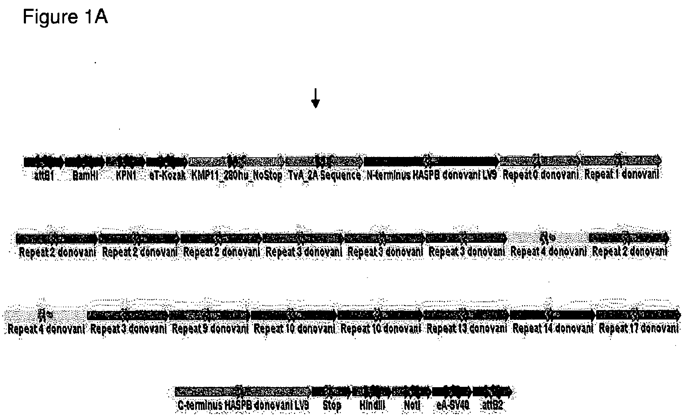

Figure 1A synthetic KMP_HASPB_{concensus} gene construct. (A) Schematic diagram of KMP_HASPB_{concensus}. (B) Protein lysates from P815 cells transduced o/n with the indicated viral constructs were examined for protein expression by SDS-PAGE and immunoblotting with KMP and HASPB specific antisera. Protein lysates from L.donovani parasites or recombinant KMP were used as positive controls;

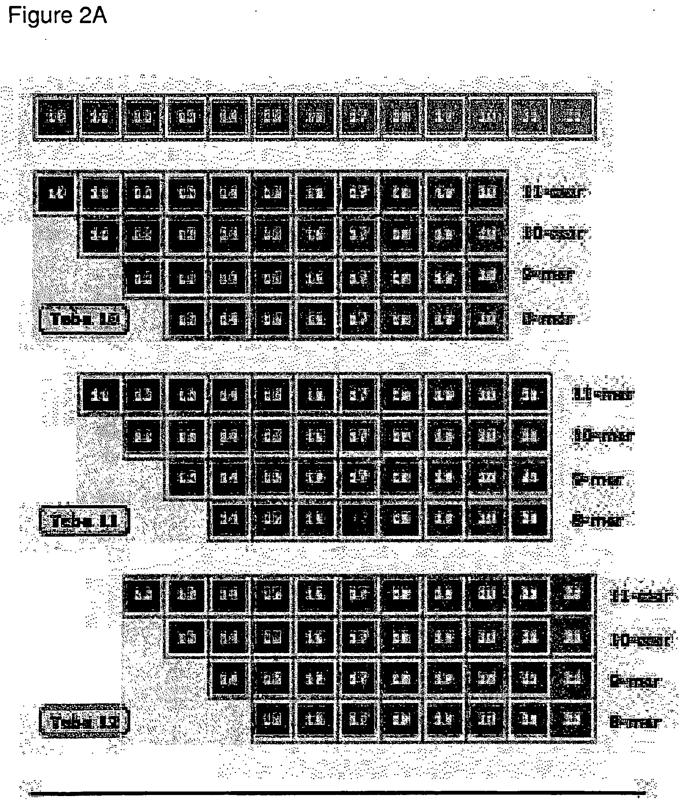

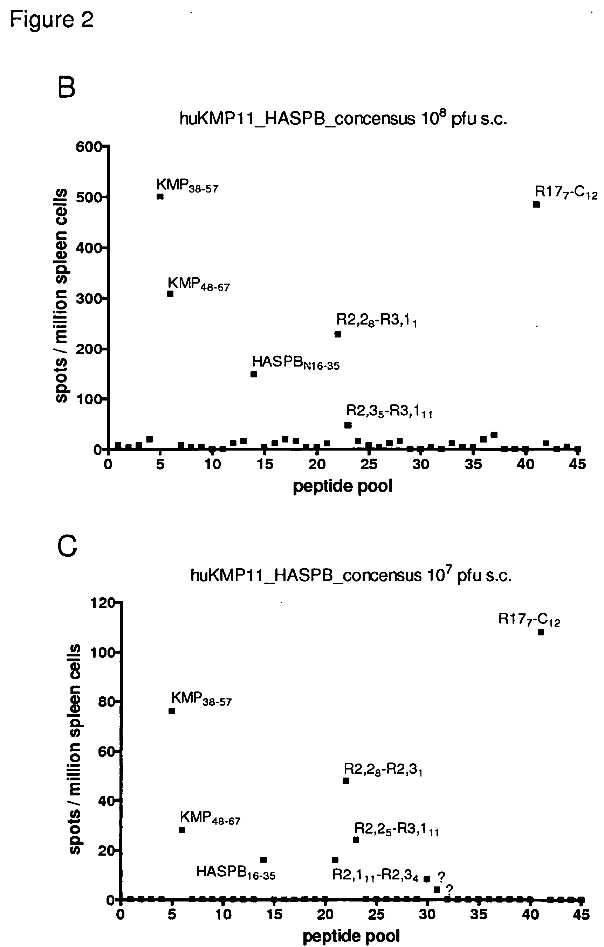

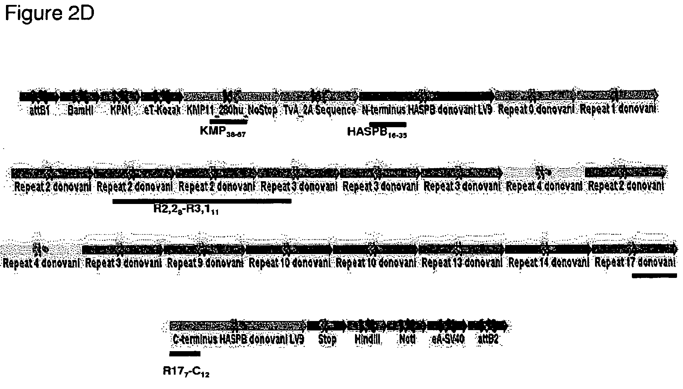

Figure 2 Antigen specific responses to KMP and HASPb proteins. (A) Schematic diagram of overlapping peptide pepset design. (B and C) Naïve BALB/c mice were vaccinated s.c with either 10⁸ PFU (B) or 10⁷ PFU (C) Ad5KMP_HASPB_{concensus}. Peptide-specific responses to KMP and HASPB proteins were assessed by ELISPOT. Splenocytes (2.5x 10⁵) were restimulated with pooled pepsets (10ug/ml). Experiments were performed at least twice using pooled splenocytes from 2 vaccinated mice. (D) A Schematic diagram of KMP_HASPB_{concensus} with identified regions of aa sequence that induce activated CD8+ T cells indicated by black bars;

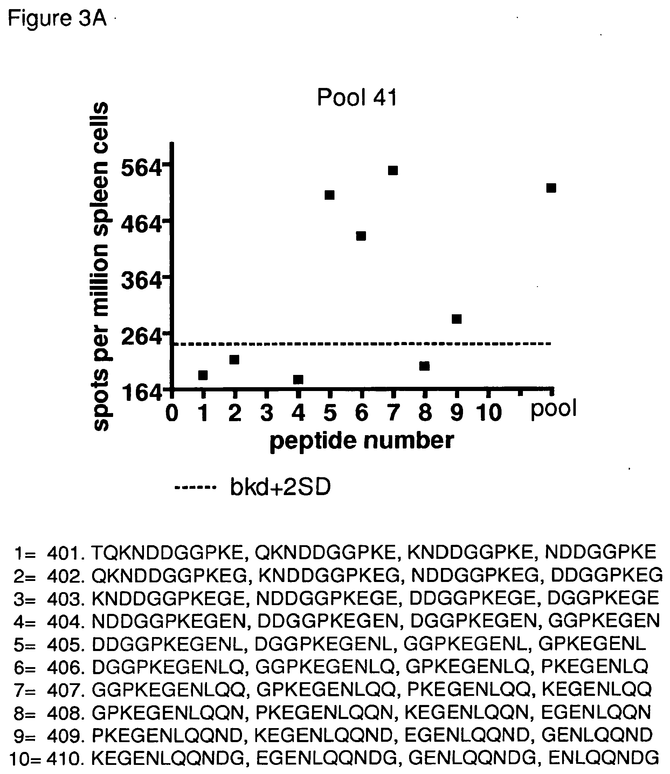

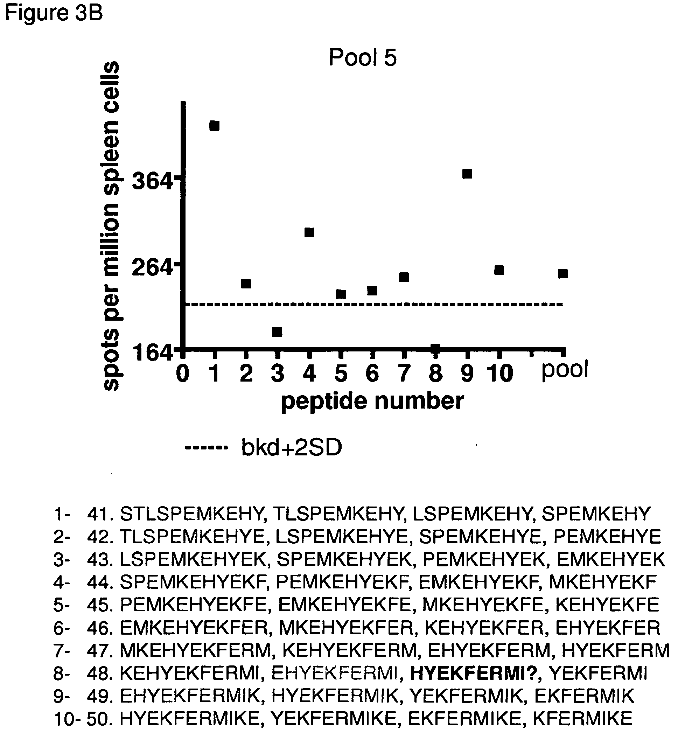

Figure 3 Fine epitope mapping of KMP and HASPB epitopes. Naïve BALB/c mice were vaccinated s.c with 10⁸ PFU Ad5-KMP_HASPB_{concensus} and 10 days later splenocytes were used in an IFNγ ELISPOT assay. Splenocytes were restimulated with individual peptide tubes used to generate HASPB pool #41 (A) and KMP pool #5 (B). Peptide sequence is also shown for each individual pepset tested with deduced epitopes shown in red text;

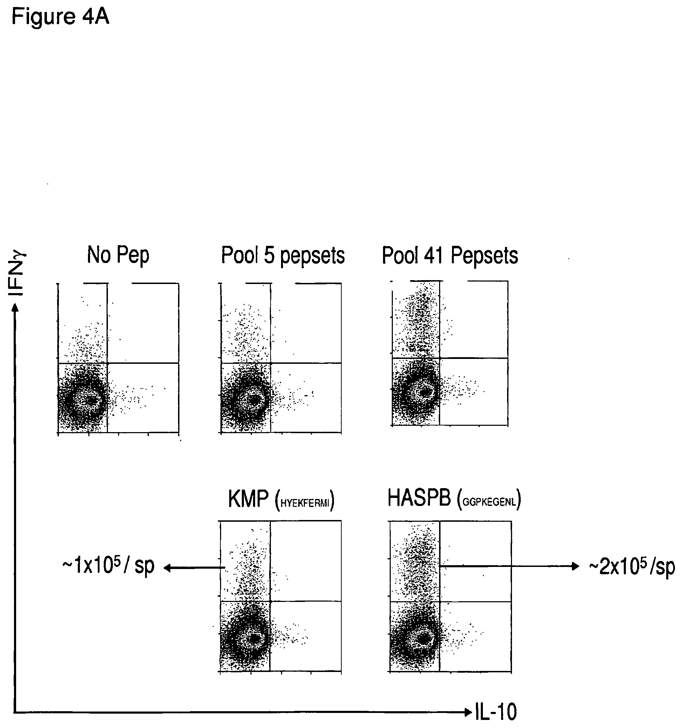

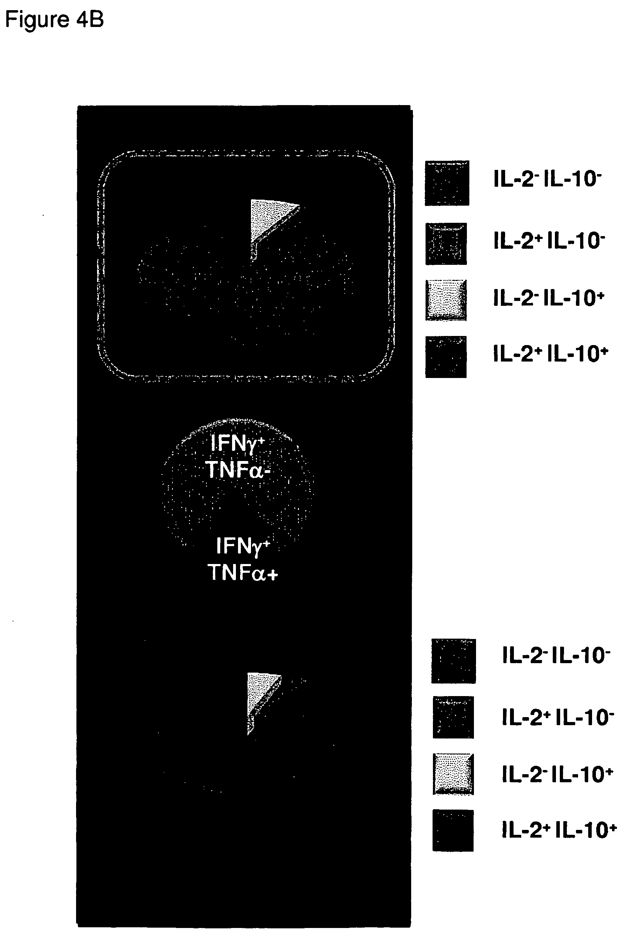

Figure 4 Verification of predicted peptide sequences. Naïve BALB/c mice were vaccinated s.c with 10⁹ PFU Ad5-KMP_HASPB_{concensus} and 10 days later splenocytes (5x106/ well) were restimulated with either pooled pepsets or synthetic pepides corresponding to predicted epitopes. Samples were restimulated for 7h and Brefeldin A was added for the last 4h of culture. Following surface staining for CD3 and CD8 samples were stained for intracellular cytokine antibodies for IFN. and IL-10 (A). (B) In a similar experiment splenic CD8+ T cells from vaccinated mice were restimulated with HASPB peptide, surface stained for CD3 and CD8, and he stained for intracellular cytokines. Pie-charts illustrate the relative contribution of the different cytokine+ T cell populations. HASPB-specific CD8+ T cells were initially separated into IFN.+TNF-or IFN.+TNF+ subsets and relative contributions of IL-2 and IL-10 was examined within each subset;

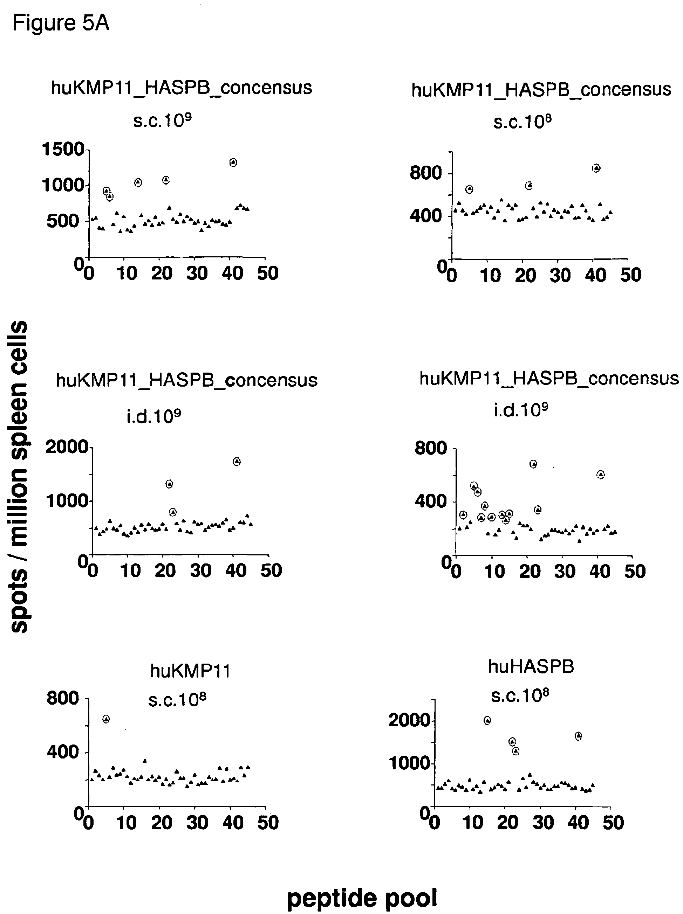

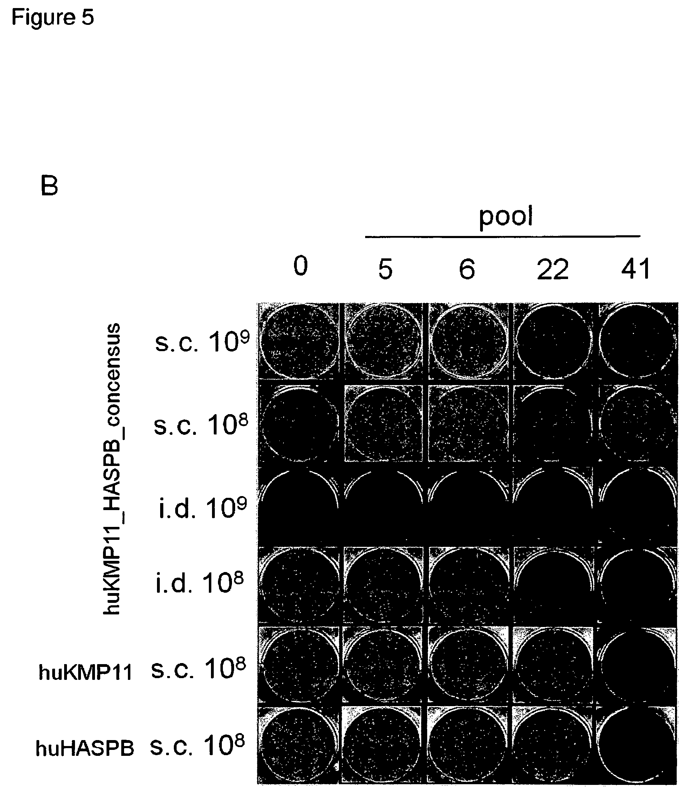

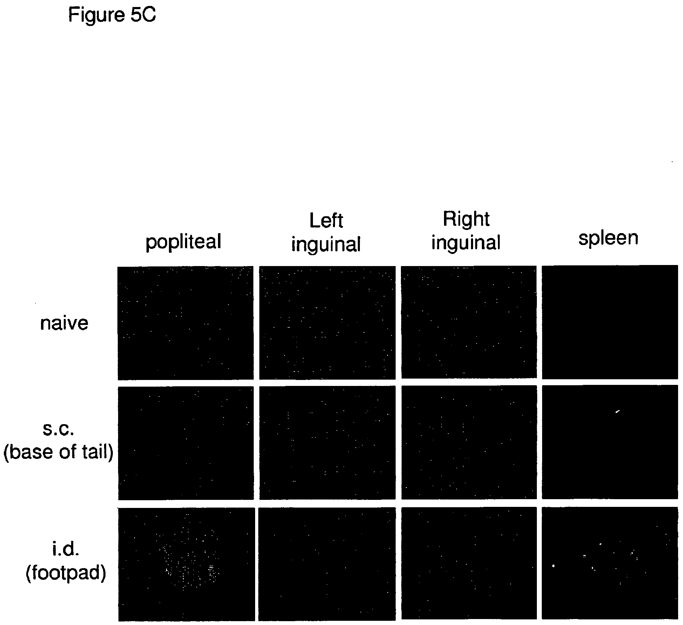

Figure 5 Intradermal vaccination induces potent immune responses to KMP_HASPB_{concensus} (A) Naïve BALB/c mice were vaccinated either s.c or i.d with Ad5KMP_HASPB_{concensus} (at either 10⁹ PFU or 10⁸ PFU). Mice were also vaccinated s.c with Ad5-KMP or Ad5-HASPBconcensus (10⁸ PFU). At 10 days post vaccination splenocytes (2.5x 10⁵) were used in IFNγ ELISPOT assay. Cells were restimulated with pooled pepsets (10ug/ml).(B) Representative images of positive wells are shown compared to stimulated controls Experiments were performed at least twice using pooled splenocytes from 2 vaccinated mice. (C) Fluorescent stereo microscopy was used to identify cellular localisation of Ad5-GFP following s.c or i.d vaccination. Naïve BALB/c mice were vaccinated either s.c or i.d with Ad-GFP for 24h and local draining LN and spleens were examined for GFP expression. LN and spleens from unvaccinated mice were used as controls;

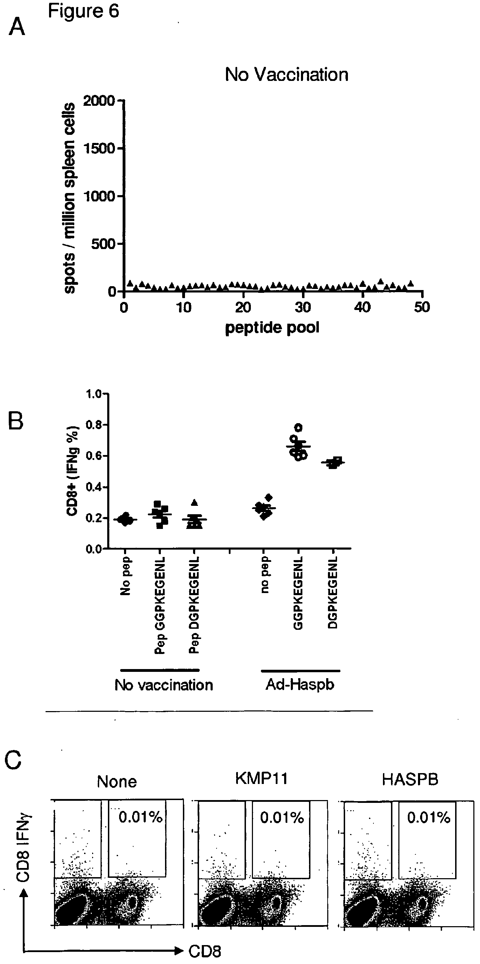

Figure 6 Lack of antigen specific CD8+ T cell responses to Ad5KMP_HASPB_{concensus} in L. donovani infected mice. (A) BALB/c mice were infected with L.donovani (3x10⁷) and at d31 splenic CD8+ T cells were analysed for responses to KMP_HASPB_{concensus} using pooled pepsets and ELISPOT assay. (B) CD8+ T cells responses in d21-infected BALB/c mice immunised with Ad5-KMP_HASPB_{concensus} were evaluated by intracellular cytokine staining using either GGPKEGENL (contained in KMP_HASPB_{concensus}) or DGPKEGENL (as found in the challenge isolate LV9). (C) BALB/c mice infected for 7 days with L. donovani were tested for CD8+ T cell responses using specific KMP and HASPB peptides;

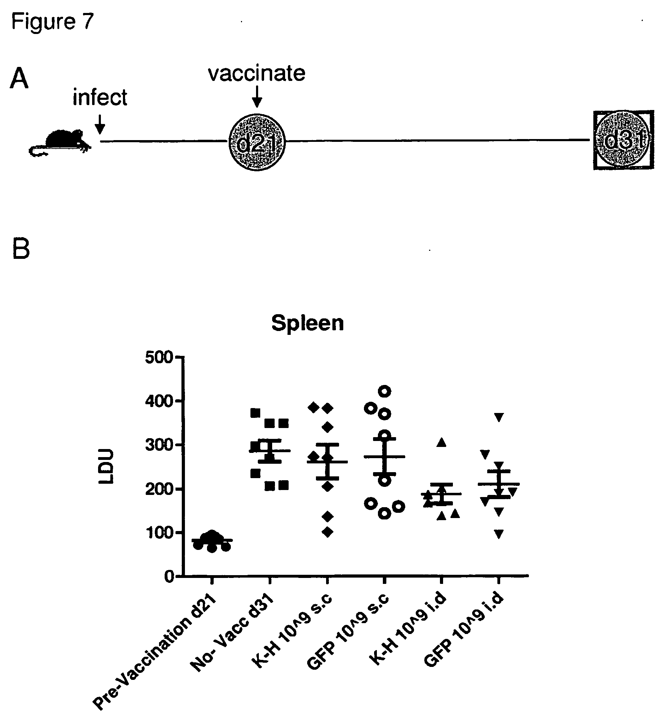

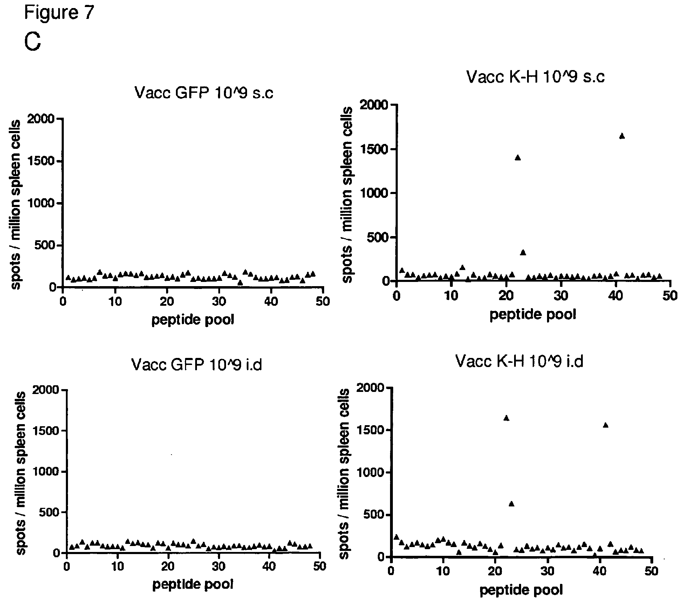

Figure 7 Comparison of the efficacy of therapeutic vaccination using Ad5KMP_HASPB_{concensus} by route of administration. (A) Schematic diagram of therapeutic vaccination strategy. (B and C) BALB/c mice were infected with L. donovani and at d21, vaccinated with 10₉ PFU Ad5KMP_HASPB_{concensus} by either the s.c or i.d route. Splenic parasite burden (B) and T cell response to pooled peptides (C) was determined in mice at d31 (d10 post vaccination). Individual mice are plotted in (A).

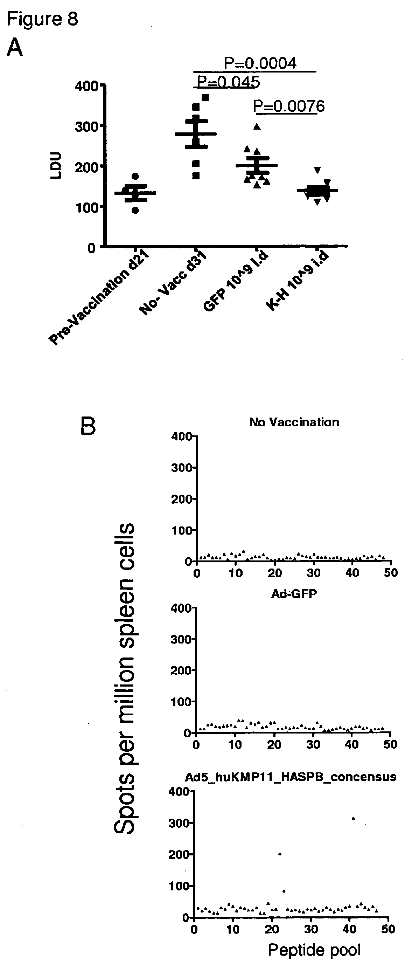

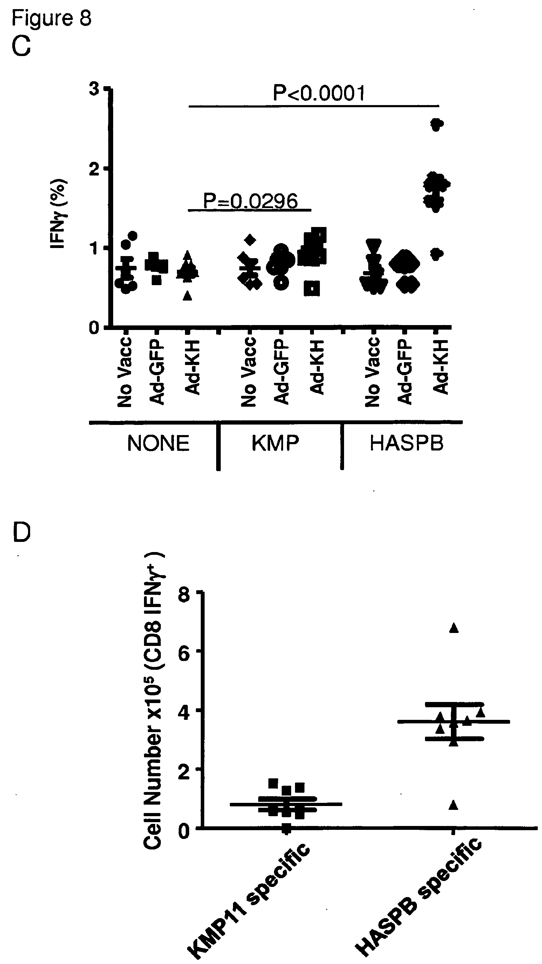

Figure 8 Therapeutic vaccination using Ad5-KMP_HASPB concensus. BALB/c mice were infected with L. donovani and at d21 p.i., immunised with 10⁹ PFU Ad5-KMP_HASPB_{concensus} by the i.d route. Parasite burden (A) and CD8+ T cell responses evaluated by ELISPOT (B). Peptide specific responses to dominant KMP and HASPB epitopes were also evaluated by intracellular cytokine staining and presented as frequency of responding CD8+T cells (C) and absolute number of specific CD8+ T cells per spleen (D). Individual mice are plotted.

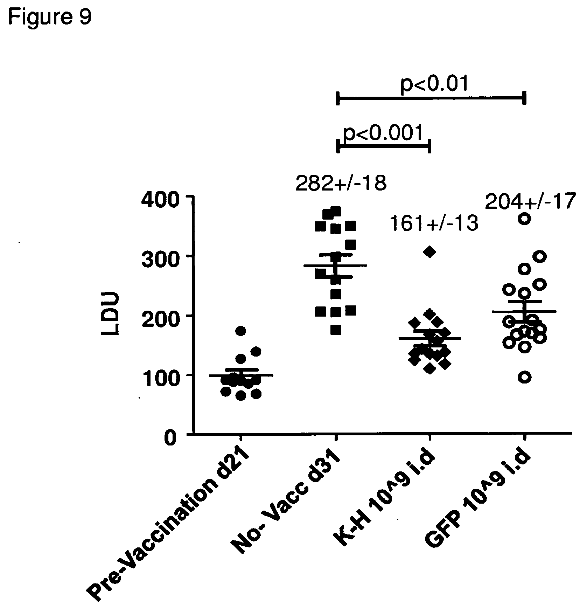

Figure 9 Therapeutic vaccination using Ad5-KMP_HASPB concensus Protection data from experiments shown in Figures 7 and 8 were combined to reach an overall estimate of the efficacy of therapeutic vaccination using Ad5KMP_HASPB_{concensus} (n=15/16 mice per group). Mean ± SE parasite burden is indicated.
Figure 10a is the nucleotide sequence of hybrid fusion of HASPB fusion gene 1 and KMP11; Figure 10b is the amino acid sequence;

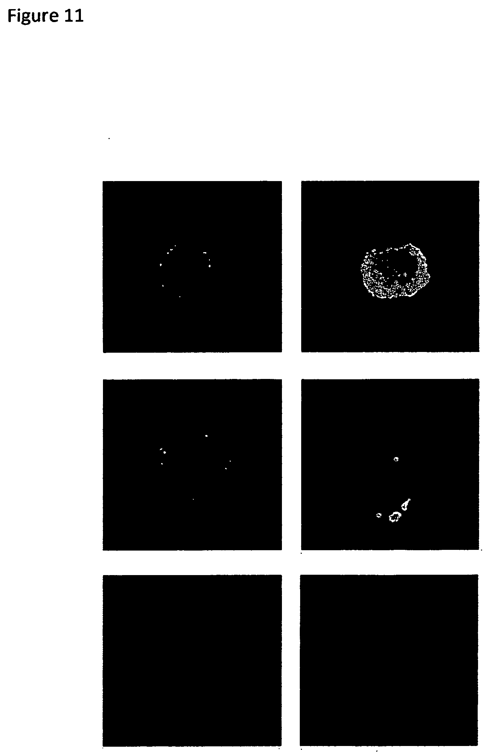

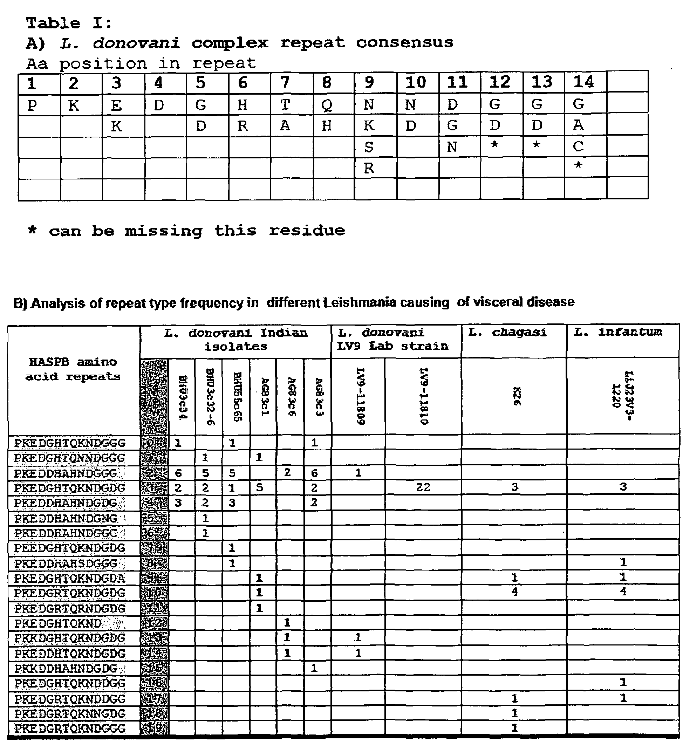

Figure 11 illustrates adenovirus transfection and expression of HASPB fusion gene.

### Materials and Methods

### Gene sequences

HASPB sequences derived from the literature, the Gene DB database, from unpublished work (D.F. Smith) and from past and present *L. donovani* isolates from Bihar (provided by P. Walden) were analysed and the diverse repeats catalogued. 19 repeats for the *L. donovani* / *infantum* complex were identified, with these 12-14 amino acid repeat units showing extensive variation at their C-termini.

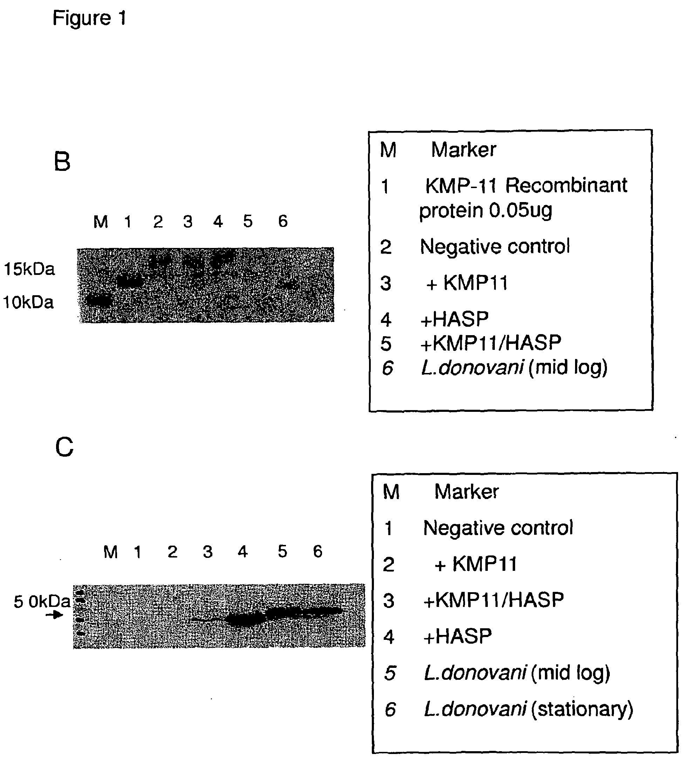

Synthetic HASPB genes were designed to encode known repeats for *L. donovani* and *L. chagasi* and also i) to preserve the relative number of repeats that are present more than once in every isolate, to maximize preservation of protein structure (= huHASPB_consensus; Figure 1) or ii) to preserve the junctional variation arising from the order of repeats found in HASPB in parasite isolates. Here all repeats are included and they are shuffled in a way to preserve the junctional sequence variation (= huHASPB_allREPEATS; Figure 2)

The resulting amino acid sequence was *'back translated'* using Genedesigner DNA2.0 software with codons optimized for human expression, selected to minimise DNA repeat structures and, importantly, manually curated. All constructs were flanked with restriction enzyme sites (for digestion and cloning into suitable vectors) as well as with both a Kozak sequence (5' of the ATG) and a SV40 derived polyadenylation sequence (3' of the termination codon) to improve translation initiation and facilitate mRNA processing respectively.

Fusion genes were also designed (Figure 3 and 4) using the synthetic KMP11-2 sequence KMP11 (KMPII-2 LinJ35_V3.2250 version GeneDB Date 09-01-2009) by linking this upstream of the synthetic hu-HASPB genes and linking the sequences with a so- called 2A sequence derived from a TetraVirus TVA (Martin Ryan, St. Andrews University pers. Comm.). All genes of interest (GOI) were custom synthesized by a commercial contractor and cloned into the plasmid vector pMA.

### Adenoviral production

GOI in pMA were cloned into an adenoviral shuttle vector and then into a E1/E3 adenoviral backbone vector. Viral packaging was performed in HEK 293 cells and primary viral stocks generated. Primary viral stock was tested for the ability to transducer P815 mastocytoma cells, to verify integrity of expression of the GOI. Viruses for vaccination studies were produced after large scale amplification of cultured cells, followed by 2 x CsCl purification and desalting. Viral titration was performed by PFU and VP. GOI may be inserted into human (e.g. Ad5, Ad35, Ad28), non-human primate (e.g. ChAd63) or simian adenoviruses.

### Immunogenicity studies

To test the immunogenicity of adenoviral vaccines expressing the GOI, C57BL/6 or BALB/c mice were immunised s.c. and / or i.p. and / or i.m with varied doses (10⁶ - 10⁸ pfu) of control and recombinant virus, once only or with a boost injection via the same or an alternate route 7 - 14 days later.

7 days after the last injection, tail blood was collected to determine the frequency of tetramer⁺ CD8⁺ T cells, using peptide tetramers based on dominant epitopes of the GOI (Rowe et al J. Virol. 2009. 83:1555-1562)

To determine in vitro recall response, spleen cell and LN suspensions were prepared using nylon mesh sieves and total splenocytes / LN cells were re-stimulated with either synthetic peptides or syngeneic cell lines (e.g. P815, EL-4) expressing the GOI. For assessment of secreted IFNγ, culture supernatants were recovered after 24h and analysed using ELISA according to standard protocols. For assessment of cytokine production by intracellular flow cytometry, splenocytes or LN cells were incubated overnight with or without peptides / transfected cell lines and then cultured in Brefeldin A for 3h before surface staining cells for CD3 and CD4 or CD3 and CD8. Cells were then fixed and permeabilised using Cytofix/Cytoperm reagents (BD Biosciences) and stained using conjugated anti-IFN-γ Ab (BD Pharmingen). Samples were analysed using a CyAan flow cytometer (Beckman Coulter).

To determine in vivo CTL activity against the GOI, syngeneic spleen cells were labelled with carboxyfluorescein diacetate succinimidyl diester (CFSE) at either a high dose (20µM ; CFSEhigh) or low dose (1µM; CFSElow). CFSEhigh cells were pulsed with peptide for 30 min at 37 °C, mixed with unpulsed CFSElow cells and then co-injected (4×107 cells i.v.) into mice previouslty immunized with recombinant adenoviruses expressing the GOI. In some instances, mice were injected with CFSEhigh-labelled syngeneic tumour cell lines (P815 or EL4) transfected with the GOI , admixed with CFSElow-labelled control transfectants. 20 h after transfer, transferred cells were recovered from the spleen, fixed and analysed by flow cytometry. The percentage of specific lysis was determined as: 1-[(%CFSEhigh infected-%CFSElow infected) / (%CFSEhigh naïve-%CFSElow naïve)]×100. (Resende et al Vaccine 2008. 26:4585-4593)

### Vaccination against Leishmania donovani

To test the efficacy of adenoviral vectors expressing the GOI as prophylactic vaccines, mice were first immunised as above. 7-28 days after the last injection, mice were challenged with 2-5 x10⁷ *L. donovani* amastigotes, prepared from the spleens of infected RAG^{-/-} mice. Mice were killed at day 14 - 56 post challenge and spleen and liver parasite burdens determined from Giemsa-stained tissue impression smears or by quantitative PCR (Depledge et al submitted; Polley et al Infect. Immun. 2006. 74:773-776; Rowe et al J. Virol. 2009. 83:1555-1562). For therapeutic vaccination, mice were first infected with *L. donovani,* as above, and then at day 14-28 post infection, injected with adenoviruses (10⁶-10⁸ pfu) expressing the GOI, using the i.m., i.p., or s.c. route. In some cases, a boost was given 7-14 days later. Mice were killed 7 days after the last injection and parasite burden determined as above.

### Epitope mapping of GOI

GOI were mapped for CD8⁺ T cell epitopes using a truncated PepSet (Mimotopes Inc) based on the predicted translated ORF, and where each nominal 11 mer peptide was represented by an equimolar mixture of the four C-terminal peptides comprising the 8mer, 9mer, 10mer and 11 mer peptides (Roederer and Koup. J. Immunol. Methods 2003. 274:221). Positive peptides were identified by IFNγ ELISPOT assy of re-stimulated spleen cells from immunised mice (above), according to standard protocols (Resende et al. Vaccine 2008. 26:4585-4593)

### Example 1

The synthetic huHASPB-concensus sequence was cloned into an Adenovirus serotype 5 (Ad5) vector and viral supernatant used to infect murine P815 mastocytoma cells. Expression of the expressed huHASPB_concensus gene product was evaluated 18h later by immunofluorescence, using a polyclonal rabbit anti-HASPB antibody. (Figure 14a-d) Antibody staining on representative cells transduced with Ad5-huHASPB_concensus; (Figure 14e-f) Antibody staining in control cells transduced with Ad5-huKMP-11.

### Example 2

### Polymorphisms in KMP11 and HASPB and epitope mapping.

KMP-11 sequences obtained from Bihar isolates sequenced so far are identical, and differ in only three amino acids from the database sequences, arguing for a homogeneous parasite population in the target area. In contrast, HASPB is known to feature stretches of 11-14 amino acid long repeats and sequences of six isolates examined predict isolate-specific variants of HASPB proteins with different numbers, compositions and orders of these repeats and several single amino acid exchanges. The extent of these variations, as analysed for this project is summarized in Table I. This sequence polymorphism is likely to generate diverse T cell epitopes and re-iteration of identical repeats may increase the relative abundance of epitopes encoded in these repeats. Collectively, these data suggest that whereas native KMP sequence is likely to be broadly representative of the parasite strains in circulation in the target population, this would not be the case for any single HASPB variant. We therefore designed a synthetic HASPB gene comprising the conserved N and C termini, bordering a region comprising 10 of the 17 different repeats (labelled Repeat 1, 2 3 etc) that we identified thus far in Leishmania spp. associated with visceral disease (Table I). The sequence and composition of the 10 repeat units was chosen to i) preserve the relative order of repeats that are present in more than just one isolate; ii) to conserve some of re-iteration of repeats found in natural isolates (e.g. strings of repeat 2); and iii) to maintain the overall protein length to that found in natural isolates. The latter was felt to be important given the peculiar structure of the HASPB molecule and a lack of predictive information on how well an extended protein might fold. This unique HASPB gene was termed HASPB_{concensus}.

### Example 3

### Construction of vaccine genes and viral vectors

In order to express both KMP11 and HASPB from a single vector, we opted to make use of the 2A sequence derived from the tetravirus TaV virus (RAEGRGSLLTCGDVEENPG) provided by Prof. M. Ryan (http://www.st-andrews.ac.uk/ryanlab/2A_2Alike.pdf). 2A sequences encode stretches of 20-30 amino acids and can be used to link two or more protein coding genes together in a single long open reading frame which allows multiple genes to be expressed under the control of a single promoter (or important in our context from a single integration site in a single vaccine vector) (de Felipe et al Gene Therapy 1999. 6:198-208). During translation the polyprotein is processed at the 2A sequence resulting in each protein being expressed as a single cleavage product. Thus, we expect KMP11 and HASPB to be expressed as individual proteins rather than as a fusion protein. This strategy avoids the generation of neo-epitopes between proteins and also allows independent protein transport through the antigen processing machinery of dendritic cells and macrophages. 2A sequences are in the public domain, have recently been used in a range of technologies (catalogued at http://www.st-andrews.ac.uk/ryanlab/page10.htm) and have been approved for use in human gene therapy by the FDA (Jones, S., et al Human Gene Therapy 2009. 20:630-640).

The final amino acid sequence was back-translated using Gene Designer DNA2.0 software using codons optimised for human expression and selected to minimise DNA repeat structures. The construct was flanked by Kozak sequence 5' of the ATG and a SV40 derived polyadenylation sequence to improve translation initiation and allow mRNA processing, respectively. The final synthetic gene is depicted in Figure 1A and was synthesised under contract by Geneart. The synthetic KMP_HASPB_{concensus} gene (as well as synthetic genes encoding only KMP11 or HASPB_{concensus}), were inserted into a E1/E3 deleted Ad5 viral vector by Vector Biolabs Inc. and experimental batches of ∼5x1012 viral particles (vp) were provided. The vp to plaque forming unit (pfu) ratio of the viruses was ∼20-25:1. Viral doses for all experiments are expressed in terms of pfu. To confirm protein expression from these viruses, we transduced the murine mastocytoma P815 with virus at a MOI of 100:1 and 24-36h later, performed cell lysis followed by western blot, using anti-KMP11 and anti-HASPB anti-sera (Figure 1B and C). Control samples included untransduced P815 cells as well as either metacyclic promastigotes of L. donovani (which highly express HASPB) or recombinant KMP11 (produced in E. coli). Of note, the levels of expression of HASPB were somewhat lower in cells transduced with bi-protein encoding Ad5-KMP_HASPB_{concensus} compared to cells transduced with Ad5-HASPB_{concensus}, suggesting that there may be less efficient expression of HASPB placed after the 2A cleavage sequence. However, this was not further investigated and may also be the result of differing levels of viral transduction between the two samples. These data confirm, however, that the synthetic genes can give rise in mammalian cells to the respective full length KMP11 and HASPB proteins.

### Example 4

### Immunogenicity and epitope mapping of synthetic vaccine gene products

To determine immunogenicity and identify potential CD8+ T cell epitopes within the vaccine proteins, we used a synthetic peptide approach. A truncated Pepset® (Mimotopes, Inc.) of 11mer peptides overlapping by 10 amino acids was generated for the entire coding sequence of KMP_HASPB_{concensus} (including the 2A sequence), and comprised of 444 individual peptide tubes (each tube containing an 11mer plus its respective truncated 10, 9 and 8 mers; Figure 2A). For initial screening, we generated pools of 10 tubes, thereby spanning the entire protein sequence in 20 amino acids segments. BALB/c mice were immunised with 107 - 109 pfu of Ad5-KMP_HASPB_{concensus} by the subcutaneous route and at day 10 post immunisation, spleen cells were removed for IFN. ELISPOT assay (Mabtech, Sweden). Briefly, total spleen cells (5x105 per well in pre-coated ELISPOT plates) were restimulated overnight with peptide pools (10µg/ml), removed by washing and then plates were developed using IFN. detection mAb. ELISPOTS were counted manually under a stereomicroscope. As shown in Figure 2B and 2C, immunisation led to a robust and immunisation dose-dependent response to Ad5KMP_HASPB_{concensus}, which was reflected by targeted recognition of a number of regions of both KMP11 and HASPB. At both 107 and 108 pfu immunising doses, a consecutive pair of peptide pools within KMP11, representing a 40 amino acid stretch (KMP38-67) was recognised by BALB/c mice under this immunisation regimen. For HASPB, recognition was targeted to a single 20mer region within the conserved N-terminal (HASPBN16-35), a 20mer stretch spanning the last repeat region and the conserved C-terminal (R177 -C12) and to a 40 amino acid stretch which spanned 3 repeats (R2.28 -R3.111). A weak recognition of a further repeat region with low dose immunisation was also observed (R2.111 - R2.34). The location of the dominant regions mapped onto the synthetic vaccine antigen sequence is shown in Figure 2D. Similar results were seen using an immunisation dose of 109 pfu, though the intensity of the response given the number of spleen cells plated made quantitation difficult (data not shown). Of note, there was no evidence from these studies in mice that the 2A sequence was recognised by CD8+ T cells, either alone or as part of a neo-epitope with KMP11 or HASPB sequence.

### Example 5

We then proceeded to fine map the two most immunodominant regions to provide defined peptides for subsequent immune monitoring and also potentially to generate tetramer probes. As shown in Figure 3A, restimulation with the 10 peptide tubes that comprised pool 41 readily identified 3 individual tubes containing truncated peptides recognised in this assay (peptides 405, 406 and 407). Searching these peptide sequences for 8, 9 or 10 mer sequences shared in all three tubes revealed only one potential 9 mer sequence (GGPKEGENL) as a putative epitope for CD8+ T cell recognition. This fine mapping strategy was not as clear for the KMP11 peptides represented by pool 5, but nevertheless, a strong candidate peptide sequence was identified (HYEKFERMI; Figure 3B). Potential as class I epitopes in BALB/c mice was confirmed using a variety of algorithms (Syfpeithi, Rankpep, PREDBALB/c and Bimas). These peptides were subsequently directly synthesised and compared in their ability to recall CD8+ T cell IFN responses with the original peptide pool by intracellular flow cytometry. As shown in Figure 4A, the two peptides faithfully recalled CD8+ T cell response to the same degree as the peptide pools from which they were identified. Furthermore, we could confirm by multi-parameter flow cytometry that immunisation with KMP_HASPB_{concensus} induced polyfunctional CD8+ T cell responses, including those producing IFN., TNF and IL-2 (Figure 4B), a combination of cytokines that has been implicated in protection in the murine model of L. major infection (Darrah et al Nat Med. 2007. 13:843-50). These data confirm GGPKEGENL and HYEKFERMI as H2d-restricted CD8+ T cell epitopes recognized following vaccination of naïve BALB/c mice with Ad5KMP_HASPB_{concensus}. Importantly, the levels of CD8+ T cell response were of the same order of magnitude as those reported for single dose vaccination in other systems (e.g. ME-TRAP of malaria; Reyes-Sandoval et al Eur J Immunol. 2008. 38:732-41) and better than reported for homologous prime -boost with Ad5-L. donovani A2 (Resende et al Vaccine. 2008 26:4585-93).

### Example 6

We next evaluated the response to Ad5-KMP_HASPB_{concensus} comparing subcutaneous vs. intradermal immunisation. As shown in Figure 5A, intradermal immunisation induced a stronger CD8+ T cell response, as measured by absolute numbers of ELISPOTs recorded, though this was more targeted towards epitopes within HASPB at high dose compared to the broader response observed using subcutaneous immunisation. Although difficult to quantify, the spot size following intradermal immunisation appeared larger than that obtained after subcutaneous immunisation, suggesting a greater potency to the IFN. response (Figure 5B). We also evaluated the distribution of Ad5-GFP in vivo after administration via thee alternate routes. As shown in Figure 5C, following subcutaneous immunisation at the base of tail, virus was distributed in both inguinal LN (and at higher magnification could be seen within macrophages and DC in the subcapsular sinus and paracortical region of the LN; data not shown). In contrast and as expected, intradermal immunisation in the footpad led to asymmetric distribution of virus in the popliteal LNs, with only the node draining the injection site demonstrable positive for virus. Of interest, following footpad immunisation, some virus was detected in the spleen and this was commensurate with a degree of splenomegaly. Nevertheless, it is difficult to directly equate these results to the likely outcome in man. For example, whereas Ad induced responses are greater in man after intramuscular than intradermal delivery, a similar situation is not seen in mouse (Hill, personal communication).

### Example 7

We next asked whether mice infected only with L. donovani had generated responses to either KMP11 or HASPB. In pilot experiments, we observed that ELISPOT assays were difficult to interpret when using whole spleen cells, due to the increased level of spontaneous IFN. produced in infected mice (from CD4+ and CD8+ T cells, NK and NKT cells). Therefore, to assess peptide-specific CD8+ recall responses, we first MACS purified CD8+ T cells from infected mice and then used uninfected spleen cells as a source of APC. As shown in Figure 6A, we failed to identify any recall CD8+ T cell response to either KMP11 or HASPB in mice infected for 31 days with L. donovani, when using the entire peptide set spanning the KMP_HASPB_{concensus}. This result was surprising given that this synthetic gene encodes for native KMP11, as found in L. donovani, the conserved N and C termini of HASPB, and 'repeat 3' from the laboratory strain of L. donovani used in these experimental infections (strain LV9). One possible explanation for the loss or absence of recognition of the dominant HASPB C terminal epitope was that within LV9, the putative position 1 of this 9mer contained a G - D substitution compared to that encoded by KMP_HASPB_{concensus}. Although position 1 has not been shown to influence recognition by H2d-restricted T cells, we synthesised the corresponding DGPKEGENL peptide and compared this with the GGPKEGENL epitope for recognition after vaccination and after infection. As shown in Figure 6B, DGPKEGENL was well recognized by CD8+ T cells recovered from mice primed by vaccination with Ad5-KMP_HASPB_{concensus} (containing GGPKEGENL). Similarly, using this peptide to recall responses in L. donovani-infected mice also failed to identify the presence of HASPB reactive CD8+ T cells (data not shown). We conclude from these data that mice infected with L. donovani do not have a detectable recall CD8+ T cell responses to either KMP11 or HASPB. To determine whether this was due to clonal exhaustion, suppression or lack of priming, we examined the early response to KMP_HASPB_{concensus} (Figure 6C). Even mice infected for only 7 days failed to respond to any of the peptides pools. We therefore believe that both KMP11 and HASPB represent weakly immunogenic antigens poorly primed during early experimental infection with L. donovani, arguing strongly in favour of using an adenoviral vector, given their strong unchallenged superiority in priming CD8+ T cell responses.

### Example 8

### Protective efficacy of KMP_HASPB_{concensus}.

Based on the above data, we progressed to evaluating the efficacy of Ad5KMP_HASPB_{concensus} as a therapeutic intervention against experimental VL. Mouse models of VL show organ specific differences in disease outcome. Whilst the liver is able to resolve infection, the spleen fails to do so and is characterised by massive splenomegaly with associated disruption to the local lymphoid tissue architecture (Kaye et al Immunol Rev. 2004. 201:239-53). We elected to study the impact of therapeutic vaccination on splenic parasite burden at the time point when this was increasing at its maximal rate and when tissue microarchitectural breakdown has already begun to occur. We adopted this approach as it provides the most stringent test to intervention. In the clinic, it is also likely, as has been done for recent immunological studies (Nylen et al J Exp Med. 2007. 204:805-17) that initial clinical trials of therapeutic vaccines will be conducted on patients with early diagnosis. Therefore, we vaccinated mice 21 days after infection with L. donovani, and assessed splenic parasite burden 10 days later. Under similar conditions, the spleen is known to be relatively refractory to drug treatment. For example, using a range of clinical and laboratory isolates of L. donovani, Carter and colleagues reported that administration of sodium stibogluconate (300mg/kg Sbv i.v.) resulted in only 11-52% suppression of splenic parasite burden (Carter et al Antimicrob. Agents Chemother. 2001. 45:3555-3559). Similarly, even multiple doses at 8mg/kg of Ambisome (liposomal amphotericin B) fail to effect > 80% reduction in splenic parasite burden (Mullen et al Antimicrob. Agents Chemother 1997. 41: 2089-2092). Partial drug indeed clearance splenic parasite load is usually followed by rapid recrudescence after termination of drug therapy (Kaye, unpublished). Based on these and similar data, we set our benchmark for effective single dose therapeutic vaccination as a suppression of splenic parasite growth of > 50%. Based on historical data indicating an increase in parasite burden of approximately 3 fold from day 21 - 28/31 and a standard deviation of approximately 25% of the mean parasite burden (expressed as Leishman Donovan Units) at d28/31, we calculated that with n=8 mice per group, we would have 90% power to detect a reduction of parasite burden at day 28/31 of 50% with a significance level of 0.05.

The vaccination schedule is summarised in Figure 7A. We first compared immunisation via the intradermal and subcutaneous route. As shown in Figure 7B, subcutaneous immunisation with either Ad5-GFP (a control virus) or Ad5-KMP_HASPB_{concensus} failed to provide any protection to mice against progressive splenic infection with L. donovani. In contrast, intradermal immunisation with Ad5-KMP_HASPB_{concensus} was significantly protective, suppressing splenic parasite growth by 48±10% (p=0.008). Control Ad5-GFP did not induce a significant level of protection (37±15%), though there was some suggestion of a weak antigen-independent protective response.

To determine whether vaccination had primed KMP11 and/or HASPB-specific CD8+ T cells in these infected mice, we conducted ELISPOT assays using purified CD8+ T cells from infected mice (with normal spleen cells acting as APC). As shown in Figure 7C, d31 infected mice vaccinated 10 days earlier with Ad5-GFP did not show any response to any of the peptide pools that spanned KMP_HASPB_{concensus}. In contrast, vaccinated mice made good responses to HASPB, equivalent to or better than those observed in naïve mice that were vaccinated with KMP_HASPB_{concensus} (Figure 2). Of note, although the protective efficacy of Ad-5-KMP_HASPB_{concensus} was immunisation route dependent, both routes induced similar levels of CD8+ T cell response to the HASPB epitopes. Surprisingly, given the clear priming of the response to HASPB, we found no recall response to KMP11 epitopes, as measured by ELISPOT, in vaccinated infected mice, irrespective of the route of immunisation, suggesting further constraints of recognition, expansion or survival of CD8+ T cells recognising this antigen.

### Example 9

We then conducted a second vaccination experiment using intradermal immunisation only. In this experiment (Figure 8A), the control of parasite burden stimulated as a result of vaccination with Ad5-KMP_HASPB_{concensus} was almost 90% (p=0.0004) compared to unvaccinated mice. Again, some level of bystander protection, probably mediated by an antiviral Type I IFN response, was observed using Ad5-GFP (p=0.045). Again, we found good priming of CD8+ T cell responses to HASPB but a lack of priming against KMP11, as determined by ELISPOT (Figure 8B). By intracellular flow cytometry (Figure 8C), a weak KMP response could be identified, which equated to ∼105 KMPHYEKFERMI-specific CD8+ T cells per spleen in vaccinated infected mice. In contrast spleens in these mice contained ∼ 4-fold more HASPBGGPKEGENL specific CD8+ T cells (Figure 8D).

### Example 10

Finally to derive an estimate of the overall protective efficacy of Ad5-KMP_HASPB_{concensus}, we pooled the data from the two experiments described above. As shown in Figure 9, our overall estimate of the efficacy of therapeutic vaccination using Ad5-KMP_HASPB_{concensus}, administered as a single 'prime' and measured as suppression of spleen parasite growth, was 74±8.5% (p=0.001 vs. unvaccinated mice). By using the lower 95% Cl of the mean parasite burden of unvaccinated mice as a cutoff value for protection, 93% (14/15) of mice vaccinated intradermally with 10⁹ pfu Ad5-KMP_HASPB_{concensus} responded with reduced parasite burden, compared to 68% (11/16) of mice vaccinated with the same dose of AD5-GFP. Ad5-KMP_HASPB_{concensus} thus constitutes an effective therapeutic intervention in this experimental model of infection.

## Claims

1. A nucleic acid molecule comprising a nucleotide sequence selected from the group consisting of:
i) a nucleic acid molecule consisting of the nucleotide sequence as represented in Figure 12a and which encodes dominant leishmanial antigens; and
iii) a nucleic acid molecule comprising nucleotide sequences that are degenerate as a result of the genetic code to the nucleotide sequence defined in (i) and and encodes an amino acid sequence as represented in Figure 12b.

2. A nucleic acid molecule according to claim 1 wherein the molecule comprises or consists of a nucleotide sequence as represented in Figure 12a.

3. A polypeptide encoded by a nucleic acid molecule according to claim 1 or 2.

4. A polypeptide according to claim 3 wherein said polypeptide comprises the amino acid sequence represented in Figure 12b.

5. A transcription cassette comprising: a nucleic acid molecule according to claim 1 or 2 operably linked to a promoter adapted for transcription of the nucleic acid molecule associated therewith.

6. A transcription cassette according to claim 5 wherein said promoter is a constitutive, regulatable, an inducible and/or tissue/cell specific promoter.

7. A transcription cassette according to claim 5 or 6 wherein said promoter is a skeletal muscle specific promoter.

8. A vector comprising a nucleic acid molecule according to claim 1 or 2 or any of claims 5-7.

9. A vaccine composition comprising a nucleic acid molecule or part thereof according to claim 1 or 2 or a vector according to claim 8.

10. A vaccine composition comprising a polypeptide or part thereof according to claim 3 or 4.

11. A vaccine composition according to claim 9 or 10 wherein said composition includes a carrier and/or optionally an adjuvant.

12. A vaccine composition according to claim 11 wherein said adjuvant is selected from the group consisting of: cytokines selected from the group consisting of GMCSF, interferon gamma, interferon alpha, interferon beta, interleukin 12, interleukin 23, interleukin 17, interleukin 2, interleukin 1, TGF, TNFα, and TNFβ.

13. A vaccine composition according to claim 11 wherein said adjuvant is a Toll Like Receptor [TLR] agonist.

14. A vaccine composition according to claim 11 wherein said TLR agonist is selected from the group consisting of: CpG oligonucleotides, flagellin, monophosphoryl lipid A, poly I:C and derivatives thereof.

15. A vaccine composition according to claim 11 wherein said adjuvant is a bacterial cell wall derivative such as muramyl dipeptide (MDP) and/or trehelosedimycolat (TDM).

## Patentansprüche

1. Nukleinsäuremolekül, umfassend eine Nukleotidsequenz, ausgewählt aus der Gruppe bestehend aus:
i) einem Nukleinsäuremolekül, bestehend aus der Nukleotidsequenz, die wie in Figur 10a dargestellt ist und dominante Leishmanien-Antigene kodiert; und
ii) einem Nukleinsäuremolekül, das Nukleotidsequenzen umfasst, die infolge des genetischen Kodes an der in (i) definierten Nukleotidsequenz degeneriert werden, und das eine wie in Figur 10b dargestellte Aminosäuresequenz kodiert.

2. Nukleinsäuremolekül nach Anspruch 1, wobei das Molekül eine wie in Figur 10a dargestellte Nukleotidsequenz umfasst oder daraus besteht.

3. Polypeptid, das durch ein Nukleinsäuremolekül nach Anspruch 1 oder 2 kodiert wird.

4. Polypeptid nach Anspruch 3, wobei das Polypeptid die in Figur 10b dargestellte Aminosäuresequenz umfasst.

5. Transkriptionskassette, umfassend: ein Nukleinsäuremolekül nach Anspruch 1 oder 2, das an einen Promotor funktionsfähig gebunden ist, der zur Transkription des damit verbundenen Nukleinsäuremoleküls geeignet ist.

6. Transkriptionskassette nach Anspruch 5, wobei der Promotor ein konstitutiver, regulierbarer, ein induzierbarer und/oder ein gewebe-/zellspezifischer Promotor ist.

7. Transkriptionskassette nach Anspruch 5 oder 6, wobei der Promotor ein skelettmuskelspezifischer Promotor ist.

8. Vektor, umfassend ein Nukleinsäuremolekül nach Anspruch 1 oder 2 oder einem der Ansprüche 5 - 7.

9. Impfstoffzusammensetzung, umfassend ein Nukleinsäuremolekül oder einen Teil davon nach Anspruch 1 oder 2 oder einen Vektor nach Anspruch 8.

10. Impfstoffzusammensetzung, umfassend ein Polypeptid oder einen Teil davon nach Anspruch 3 oder 4.

11. Impfstoffzusammensetzung nach Anspruch 9 oder 10, wobei die Zusammensetzung einen Träger und/oder wahlweise einen Hilfsstoff einschließt.

12. Impfstoffzusammensetzung nach Anspruch 11, wobei der Hilfsstoff ausgewählt ist aus der Gruppe bestehend aus: Cytokinen, ausgewählt aus der Gruppe bestehend aus GMCSF, Interferon-gamma, Interferon-alpha, Interferon-beta, Interleukin-12, Interleukin-23, Interleukin-17, Interleukin-2, Interleukin-1, TGF, TNFα und TNFβ.

13. Impfstoffzusammensetzung nach Anspruch 11, wobei der Hilfsstoff ein Toll-Like-Rezeptor-[TLR]-Agonist ist.

14. Impfstoffzusammensetzung nach Anspruch 11, wobei der TLR-Agonist ausgewählt ist aus der Gruppe bestehend aus: CpG-Oligonukleotiden, Flagellin, Monophosphoryllipid A, Poly-I:C und Derivaten davon.

15. Impfstoffzusammensetzung nach Anspruch 11, wobei der Hilfsstoff ein Bakterienzellwandderivat wie Muramyldipeptid (MDP) und/oder Trehalosedimycolat (TDM) ist.

## Revendications

1. Molécule d'acide nucléique comprenant une séquence nucléotidique choisie dans le groupe constitué de :
i) une molécule d'acide nucléique constituée de la séquence nucléotidique telle que représentée sur la figure 10a et qui code pour des antigènes leishmaniens dominants ; et
ii) une molécule d'acide nucléique comprenant des séquences nucléotidiques qui sont dégénérées en conséquence du code génétique en la séquence nucléotidique définie en (i) et qui code pour une séquence d'acides aminés telle que représentée sur la figure 10b.

2. Molécule d'acide nucléique selon la revendication 1, dans laquelle la molécule comprend ou est constituée d'une séquence nucléotidique telle que représentée sur la figure 10a.

3. Polypeptide codé par une molécule d'acide nucléique selon la revendication 1 ou 2.

4. Polypeptide selon la revendication 3, dans lequel ledit peptide comprend la séquence d'acides aminés représentée sur la figure 10b.

5. Cassette de transcription comprenant : une molécule d'acide nucléique selon la revendication 1 ou 2 liée fonctionnellement à un promoteur adapté pour la transcription de la molécule d'acide nucléique qui y est associée.

6. Cassette de transcription selon la revendication 5, dans laquelle ledit promoteur est un promoteur constitutif, régulable, inductible et/ou spécifique du tissu/de la cellule.

7. Cassette de transcription selon la revendication 5 ou 6, dans laquelle ledit promoteur est un promoteur spécifique du muscle squelettique.

8. Vecteur comprenant une molécule d'acide nucléique selon la revendication 1 ou 2 ou l'une quelconque des revendications 5 à 7.

9. Composition vaccinale comprenant une molécule d'acide nucléique ou une partie de celle-ci selon la revendication 1 ou 2 ou un vecteur selon la revendication 8.

10. Composition vaccinale comprenant un polypeptide ou une partie de celui-ci selon la revendication 3 ou 4.

11. Composition vaccinale selon la revendication 9 ou 10, dans laquelle ladite composition comprend un support et/ou éventuellement un adjuvant.

12. Composition vaccinale selon la revendication 11, dans laquelle ledit adjuvant est choisi dans le groupe constitué de : cytokines choisies dans le groupe constitué du GM-CSF, de l'interféron gamma, de l'interféron alpha, de l'interféron bêta, de l'interleukine 12, de l'interleukine 23, de l'interleukine 17, de l'interleukine 2, de l'interleukine 1, du TGF, du TNFα, et du TNFβ.

13. Composition vaccinale selon la revendication 11, dans laquelle ledit adjuvant est un agoniste de récepteur de type Toll [TLR].

14. Composition vaccinale selon la revendication 11, dans laquelle ledit agoniste de TLR est choisi dans le groupe constitué de : oligonucléotides à CpG, flagelline, monophosphoryl-lipide A, poly I:C et leurs dérivés.

15. Composition vaccinale selon la revendication 11, dans laquelle ledit adjuvant est un dérivé de la paroi de la cellule bactérienne tel que le muramyldipeptide (MDP) et/ou le dimycolate de tréhalose (TDM).
